(19) Europäisches Patentamt
European Patent Office
Office européen des brevets

(11) Publication number: **0 199 530**
**B1**

(12) # EUROPEAN PATENT SPECIFICATION

(45) Date of publication of patent specification: **27.06.90**

(51) Int. Cl.$^5$: **C 07 C 67/39,** C 07 C 69/54

(21) Application number: **86302818.9**

(22) Date of filing: **16.04.86**

(54) **Process for one step esterification using an intermetallic palladium based catalyst system.**

(30) Priority: **18.04.85 US 724533**

(43) Date of publication of application:
**29.10.86 Bulletin 86/44**

(45) Publication of the grant of the patent:
**27.06.90 Bulletin 90/26**

(84) Designated Contracting States:
**AT BE CH DE FR GB IT LI LU NL SE**

(56) References cited:
**EP-A-0 089 587**
**EP-A-0 117 496**
**GB-A-2 070 601**

(73) Proprietor: **THE STANDARD OIL COMPANY**
**200 Public Square, 36-F-3454**
**Cleveland Ohio 44114 (US)**

(72) Inventor: **Paparizos, Christos**
**28748 Forest Road**
**Willowick Ohio 44094 (US)**
Inventor: **Shaw, Wilfrid Garside**
**1028 Linden Lane**
**Lyndhurst Ohio 44124 (US)**
Inventor: **Callahan, James Louis**
**Star Route, Box 363 AA**
**Norwich, New York 13815 (US)**

(74) Representative: **Smith, Sydney et al**
**Elkington and Fife Beacon House 113 Kingsway**
**London WC2B 6PP (GB)**

**EP 0 199 530 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Technical field

Many different processes, including those that are two-step, together with varied catalyst systems, have been utilized heretofore to esterify aldehydes such as, for example, methacrolein with alcohols.

The present invention is directed to an esterification process for the direct one-step conversion of aldehydes and alcohols to the corresponding ester, this process being one conducted in the presence of oxygen and with a palladium based intermetallic catalyst.

### Background art

U.S. Pat. No. 4,107,204 provides a three step process for the oxidation of propylene to acrylic acid in the presence of a palladium catalyst and acetic acid. The catalyst employed is a supported palladium-copper or palladium-silver two metal compound.

U.K. Pat. No. 1,433,168, owned by the Assignee of record, is directed toward a one step process for the preparation of acrylate and methacrylate esters from unsaturated aldehydes over known catalysts comprising molybdenum, vanadium and tungsten. The feature of the invention is the addition of alcohol directly to the oxidation reaction, so that it is present with at least some of the aldehyde.

British Pat. No. 2,051,056 discloses a method for preparing carboxylic esters by reacting an aldehyde with an alcohol in the presence of a catalyst comprising palladium and bismuth and optionally an alkali metal or alkaline earth metal or other metal. The catalyst is prepared by chemically reducing a soluble palladium salt to palladium metal while the bismuth is present either in a metallic state or as a bismuth compound such that the two components interact with each other.

The foregoing methods, although relevant to the process of the present invention, would not lead one skilled in the art to the process of this invention, which employs specific palladium based catalysts, and the significant advantages resulting from the practice thereof.

### Disclosure of the invention

The present invention provides a process for the one step esterification of aldehydes and alcohols comprising the steps of; combining an aldehyde with an alcohol, in the presence of oxygen and in contact with an intermetallic palladium based catalyst having the general formula

$$PdTe_aZn_d$$

where a and d are from 0.25 to 3.

These catalysts can be used unsupported or supported. The operating temperature can range from 20°C to 200°C, with the preferred range being 35°C to 80°C. The oxygen pressure can range from subatmospheric to 25 atm (1961 kPa), with the preferred range being atmospheric to 10 atm (98 kPa). The alcohol to aldehyde ratio can range from 1:1 to 50:1 with the preferred range being 2 to 20:1.

### Preferred mode for carrying out the invention

In the practice of this invention, an aldehyde such as acrolein, butyraldehyde or methacrolein is converted to its corresponding ester, in the presence of oxygen, with an alcohol such as methanol or ethanol, together with the intermetallic palladium based catalyst system utilized in the practice of this invention. The invention process is a one-step process that produces esters under mild conditions and achieves a significant per pass conversion percent.

Other aldehydes that can be employed include saturated aliphatic aldehydes having between 1 to 12 carbon atoms and preferably 1 to 6 and unsaturated aldehydes 3 to 12 carbon atoms including aromatic species. Similarly, as to the alcohols, aliphatics having from 1 to 8 carbon atoms, preferably 1 to 3 can be employed as well as diols, unsaturated alcohols and aromatic alcohols.

Besides methacrolein, other aldehydes can be used, such as saturated aliphatic aldehydes, or alpha, beta-unsaturated aliphatic aldehydes including acrolein, crotonaldehyde and the like. Finally, aromatic aldehydes such as benzaldehyde can also be used. Alcohols other than methyl, such as ethyl, propyl, ethylene glycol, alkyl, benzyl and the like can also be used very effectively in the esterification process.

The conversion reaction can be conducted in a closed reaction vessel by combining the catalyst and reactants, alcohol and aldehyde, together and mixing for a period of time as has been exemplified hereinbelow. Alternatively, as will be appreciated by those skilled in the art, the reaction could also be conducted in either a fixed-bed or fluid-bed reactor at temperatures of from 20°C to 200°C and pressures of 0.2 to 10 atmospheres (19 to 98 kPa). The catalyst can be employed unsupported or supported on a carrier; suitable support materials include silica, alumina, boron-phosphate, titania zirconia and the like and preferably Alundum as well as mixtures thereof. Alundum is a registered trademark of the Norton Co. for fused-alumina refractory materials. The catalyst can have any of the conventional fixed-bed forms such as coated, tablet, pellet, extruded, spherical, or fluid-bed forms such as microspherical or other forms known in the art. Presence of the catalyst increases the rate and percent of conversion per single pass.

Catalyst preparation is not a feature of the present invention but is described in detail in US—A—4,623,635 the subject matter of which is incorporated herein by reference.

The effectiveness of the catalyst system disclosed herein and its use to esterify aldehydes, such as methacrolein, with alcohols, was determined and calculated by measuring the percent per pass yield as follows:

$$\text{Percent single pass yield} = \frac{\text{Moles of product recovered}}{\text{Moles of aldehyde fed}} \times 100$$

The esterification procedure utilized in the practice of this invention has collective advantages that are significant. A solid catalyst system which is readily handled and recovered from the produced ester is utilized; the invention process is effective under mild conditions of temperature, pressure and time of reaction; good percent per pass conversion in a direct one-step procedure is also achieved.

Catalyst preparation

The palladium based catalyst that can be utilized in the practice of the esterification process of this invention can be prepared as follows:

Palladium chloride (0.886 g) was placed in a 300 cc beaker and 50 cc diluted Hcl (3.0 cc) was added. The suspension was heated to 60°C for 30 minutes after which all of the palladium chloride was dissolved. Zinc nitrate as [$zn(NO_2) \cdot 6H_2O$] (0.373 g) in 5 cc of $H_2O$ was added to the palladium solution. Then $TeO_2$ (0.199 g) was added and the mixture was stirred for 15 minutes. After that period of time, 10 cc of formalin was added to the solution. Diluted KOH solution was added to adjust the pH to 7.5. A black precipitant was formed. The mixture was stirred for 1 hour before filtration. The precipitant was washed with 1.0 liter of water, and was dried in the oven at 110°C to 120°C overnight.

Control

As a control, the preferred mode of Example 1 was repeated utilizing palladium metal as the sole catalyst.

TABLE I
Esterification of Methacrolein with palladium based catalysts

| Example No. | Catalyst metallic composition | Per pass conversion to methylmethacrylate (MMA) |
|---|---|---|
| 1 | $Pd_{1.0}Zn_{0.25}Te_{0.25}$ | 50.5 percent |
| Control | Pd° | 5.6 percent |

From the results presented in Table I, it can be seen that the catalyst according to Example 1 containing palladium, tellurium and zinc, performed very well.

Based upon the yields of methyl methacrylate that have been obtained when a palladium based catalyst has been employed according to the process of the present invention as set fourth herein, it should be apparent that a one step esterification process disclosed herein is applicable, in general, to palladium catalysts according to the invention.

**Claims**

1. A process for the one step esterification of aldehydes and alcohols comprising the steps of: combining an aldehyde with an alcohol, in the presence of oxygen and in contact with an intermetallic palladium based catalyst having the general formula

$$PdTe_aZn_d$$

where a and d are from 0.25 to 3.

2. A process as claimed in claim 1, characterised in that said aldehydes are selected from the group consisting of saturated aldehydes having from one to 12 carbon atoms and unsaturated aldehydes having from three to 12 carbon atoms including aromatic aldehydes.

3. A process as claimed in claim 1 or claim 2, characterised in that alcohols are selected from the group consisting of aliphatics having from one to 8 carbon atoms; diols, unsaturated alcohols and aromatic alcohols.

4. A process as claimed in claim 1, characterised in that the aldehyde is acrolein and said alcohol is methanol.

5. A process as claimed in claim 1, characterised in that the aldehyde is methacrolein and said alcohol is methanol.

6. A process as claimed in claim 5, characterized in that the catalyst is $PdTe_{0.25}Zn_{0.25}$.

7. A process as claimed in claim 1, characterised in that the aldehyde is acrolein, the alcohol is methanol and the catalyst is $PdTe_{0.25}Zn_{0.25}$.

8. A process as claimed in any of claims 1 to 7, characterised in that the esterification is conducted at a temperature of from 20°C to 200°C and an oxygen pressure ranging from subatmospheric to 20 atmospheric (1961 kPa).

9. A process as claimed in any of claims 1 to 8, characterised in that the alcohol is combined with the aldehyde in a ratio of from 1:1 to 50:1.

**Patentansprüche**

1. Verfahren zur Veresterung von Aldehyden und Alkoholen in einer Stufe, umfassend die Schritte der Kombination eines Aldehyds mit einem Alkohol in Anwesenheit von Sauerstoff und in Kontakt mit einem intermetallischen Katalysator auf Palladium-Basis mit der allgemeinen Formel

$$PdTe_aZn_d$$

worin a und d von 0,25 bis 3 sind.

2. Verfahren nach Anspruch 1, dadurch gekennzeichnet, daß die Aldehyde ausgewählt werden aus der Gruppe von gesättigten Aldehyden mit einem bis 12 Kohlenstoffatomen und ungesättigten Aldehyden mit drei bis 12 Kohlenstoffatomen, einschließlich aromatischer Aldehyde.

3. Verfahren nach Anspruch 1 oder Anspruch 2, dadurch gekennzeichnet, daß die Alkohole ausgewählt werden aus der Gruppe von Aliphaten mit einem bis 8 Kohlenstoffatomen; Diolen, ungesättigten Alkoholen und aromatischen Alkoholen.

4. Verfahren nach Anspruch 1, dadurch gekennzeichnet, daß der Aldehyd Acrolein ist und der Alkohol Methanol ist.

5. Verfahren nach Anspruch 1, dadurch gekennzeichnet, daß der Aldehyd Methacrolein ist und der Alkohol Methanol ist.

6. Verfahren nach Anspruch 5, dadurch gekennzeichnet, daß der Katalysator $PdTe_{0.25}Zn_{0.25}$ ist.

7. Verfahren nach Anspruch 1, dadurch gekennzeichnet, daß der Aldehyd Acrolein ist, der Alkohol Methanol ist und der Katalysator $PdTe_{0.25}Zn_{0.25}$ ist.

8. Verfahren nach einem der Ansprüche 1 bis 7, dadurch gekennzeichnet, daß die Veresterung bei einer Temperatur von 20°C bis 200°C und einem Sauerstoffdruck im Bereich von subatmosphärischem bis 20 Atmosphären (1961 kPa) durchgeführt wird.

9. Verfahren nach einem der Ansprüche 1 bis 8, dadurch gekennzeichnet, daß der Alkohol mit dem Aldehyd in einem Verhältnis von 1:1 bis 50:1 kombiniert wird.

**Revendications**

1. Procédé pour l'estérification en une étape d'aldéhydes et d'alcools comportant les étapes consistant à: combiner un aldéhyde avec un alcool, en présence d'oxygène et en contact avec un catalyseur intermétallique à base de palladium ayant la formule générale:

$$PdTe_aZn_d$$

où a et d valent de 0,25 à 3.

2. Procédé tel que revendiqué dans la revendication 1, caractérisé en ce que lesdits aldéhydes sont choisis dans le groupe constitué par les aldéhydes saturés ayant de 1 à 12 atomes de carbone y compris les aldéhydes aromatiques.

3. Procédé tel que revendiqué dans la revendication 1 ou 2, caractérisé en ce que les alcools sont choisis dans le groupe constitué d'alcools aliphatiques ayant de 1 à 8 atomes de carbone; de diols, d'alcools insaturés et d'alcools aromatiques.

4. Procédé tel que revendiqué dans la revendication 1, caractérisé en ce que l'aldéhyde est l'acroléine et ledit alcool est le méthanol.

5. Procédé tel que revendiqué dans la revendication 1, caractérisé en ce que l'aldéhyde est la méthacroléine et ledit alcool est le méthanol.

6. Procédé tel que revendiqué dans la revendication 5, caractérisé en ce que le catalyseur est $PdTe_{0,25}Zn_{0,25}$.

7. Procédé tel que revendiqué dans la revendication 1, caractérisé en ce que l'aldéhyde est l'acroléine, l'alcool est le méthanol et le catalyseur est $PdTe_{0,25}Zn_{0,25}$.

8. Procédé tel que revendiqué dans l'une quelconque des revendications 1 à 7, caractérisé en ce que l'estérification est conduite à une température comprise entre 20°C et 200°C et à une pression d'oxygène comprise entre une pression subatmosphérique et 20 atmosphères (1961) kPa).

9. Procédé tel que revendiqué dans l'une quelconque des revendications 1 à 8, caractérisé en ce que l'alcool est combiné avec l'aldéyde dans un rapport compris entre 1:1 et 50:1.